# EUROPEAN PATENT APPLICATION

(11) **EP 4 678 659 A1**
(43) Date of publication of application: **14.01.2026**
(21) Application number: 24759572.1
(22) Date of filing: 06.02.2024
(51) Int. Cl.: C07K 16/24, C12N 15/13, A61K 39/395, A61P 37/02, A61P 11/06

(54) **MONOCLONAL ANTIBODY PREPARATION TARGETING TSLP**

(30) Priority: 23.02.2023 CN 202310159488
(71) Applicant: Sichuan Kelun-Biotech Biopharmaceutical Co., Ltd., Chengdu, Sichuan 611138 (CN); Harbour Biomed (Shanghai) Co., Ltd, Shanghai 201203 (CN)
(72) Inventor: LUO, Liangbo, Chengdu, Sichuan 611138 (CN); YANG, Liu, Chengdu, Sichuan 611138 (CN); LOU, Ruyun, Chengdu, Sichuan 611138 (CN); SHAN, Wei, Chengdu, Sichuan 611138 (CN); YANG, Tong, Chengdu, Sichuan 611138 (CN); CHEN, Yan, Chengdu, Sichuan 611138 (CN); GE, Junyou, Chengdu, Sichuan 611138 (CN); TAN, Xiangyang, Chengdu, Sichuan 611138 (CN); TAN, Miao, Chengdu, Sichuan 611138 (CN)
(74) Representative: Cooley (UK) LLP
(86) International application number: PCT/CN2024/076303
(87) International publication number: WO 2024/174895

(57) **Abstract**

A monoclonal antibody preparation targeting thymic stromal lymphopoietin (TSLP). A pharmaceutical composition containing a TSLP antibody or an antigen-binding fragment thereof, comprising: 50-400 mg/mL of the TSLP antibody or the antigen-binding fragment thereof, and an adjuvant. A preparation and kit containing the pharmaceutical composition, and a use of the preparation and kit in treatment of allergic inflammation or autoimmune diseases.

## Description

The present application is based on an application with CN application number 202310159488.1 filed on 23 February 2023**,** and claims priority thereof, wherein the disclosure of this **CN** application is incorporated herein by reference in its entirety.

### Technical Field

The present application relates to the field of pharmaceutical preparations, and specifically relates to a monoclonal antibody preparation targeting **TSLP.**

### Background

Thymic stromal lymphophopietin **(TSLP)** is an inflammatory cytokine similar to IL7. The **TSLP** responds to microorganisms, physical damages, or inflammatory cytokines (e.g., **IL-Iβ** and **TNF),** is mainly secreted by epithelial cells of, e.g., skin, lung, thymus, and gastrointestinal tract, and may also be secreted under pathological conditions such as inflammation, by, e.g., stromal cells, keratinocytes, dendritic cells **(DCs),** and mast cells. The **TSLP** plays an important role in initial triggering of allergic and adaptive airway inflammations. Compared to healthy controls, the TSLP is highly expressed in airways of asthmatic patients, and its level is directly associated with the expression of **TH2** cytokines and chemokines, as well as the severity of the disease. The **TSLP** can induce the maturation of dendritic cells **(DCs),** and upregulate the expression of **OX40L.** The interaction of OX40-OX40L is involved in the polarization of **TH2** cells induced by initial **T** cells. After differentiation, it releases cytokines such as **IL-4, IL-5,** and **IL-13,** thereby resulting in mast cells, eosinophil infiltration, a series of allergic inflammatory responses, pathological changes of the airway, and asthmatic attack. The **TSLP** can effectively activate mast cells and natural killer **T (NKT)** cells, produce **TH2** cytokines such as **IL-13,** and aggravate the occurrence and development of airway inflammation.

A **TSLP** receptor is a heterodimer receptor complex composed of **IL-7Rα** and unique **TSLPR** chain **(CRFL2).** The binding of the TSLP heterodimer receptor causes the **STATS** activation and cell proliferation. The **DC** cells have highly expressed **TSLPR** and **IL-7Rα.**

WO2021115240A1 discloses a fully human antibody that can bind human TSLP. This antibody has a high affinity to TSLP, and an ability to effectively block the proliferation effect of **TSLP** on **Ba/F3** cells, block the activation of **TSLP** on **PBMC,** and secrete cytokines.

Preparations of a therapeutic protein such as an antibody, particularly a high-concentration preparation, are faced with technical challenges due to problems such as stability. An object of the present invention is to provide a high-concentration preparation of the above **TSLP** antibody with high stability.

### Summary of the Invention

In a first aspect, the present application provides a pharmaceutical composition containing a **TSLP** (thymic stromal lymphopoietin) antibody or an antigen-binding fragment thereof, comprising **50-400 mg/mL** of the **TSLP** antibody or the antigen-binding fragment thereof, and an adjuvant.

In some embodiments, the **TSLP** antibody or the antigen-binding fragment thereof comprises complementarity determining regions **(CDRs)** of:
**CDR-H1** or a variant thereof, **CDR-H2** or a variant thereof, and **CDR-H3** or a variant thereof comprised in a heavy chain variable region (VH) set forth in SEQ ID NO: 12; and/or
**CDR-L1** or a variant thereof, **CDR-L2** or a variant thereof, and **CDR-L3** or a variant thereof comprised in a light chain variable region **(VL)** set forth in **SEQ ID NO:13.**

In some embodiments, the variant has, compared to **CDR** from which the variant is derived, substitution, deletion, or addition of one or several amino acids (for example, substitution, deletion, or addition of **1, 2,** or **3** amino acids). In some embodiments, the substitution is conservative substitution.

In some embodiments, the **TSLP** antibody or the antigen-binding fragment thereof comprises:
**(I-1) VH** and/or **VL,** as defined in an **IMGT** numbering system, the **VH** comprising **CDR-H1** with a sequence **of SEQ ID NO:1,** CDR-H 2 with a sequence **of SEQ ID NO:2,** and CDR-H3 with a sequence of **SEQ ID NO:3;** and/or
the **VL** comprising **CDR-L1** with a sequence of **SEQ ID NO:4,** CDR-L2 with a sequence of **SEQ ID NO:5,** and **CDR-L3** with a sequence of **SEQ ID NO:6;**
(**I-2**) **VH** and/or **VL,** as defined in an **AbM** numbering system, the **VH** comprising **CDR-H1** with a sequence of **SEQ ID NO:7,** CDR-H2 with a sequence of **SEQ ID NO:8,** and CDR-H3 with a sequence of **SEQ ID NO:9;** and/or
the **VL** comprising **CDR-L1** with a sequence of **SEQ ID NO:10,** CDR-L2 with a sequence **of SEQ ID NO:11,** and **CDR-L3** with a sequence of **SEQ ID NO:6;**
   or
**(I-3) VH** and/or **VL,** compared to the **VH** and/or the **VL** described in any one of **(I-1)** or (**I-2**), at least one **CDR** comprising a mutation, wherein the mutation is substitution, deletion, or addition of one or several amino acids, or any combination thereof (e.g., substitution, deletion, or addition of **1, 2,** or **3** amino acids, or any combination thereof).

In some embodiments, the substitution is conservative substitution.

In some embodiments, the **TSLP** antibody or the antigen-binding fragment thereof binds to human **TSLP** and/or monkey **TSLP.**

In some embodiments, the **TSLP** antibody or the antigen-binding fragment thereof comprises:
(**II-1**) **VH** set forth in **SEQ ID NO:12** and/or **VL** set forth in any one of **SEQ ID NO:13;**
**(II-2),** having **VH** with at least **70%,** at least **80%,** at least **85%,** at least **90%,** at least **91%,** at least **92%,** at least **93%,** at least **94%,** at least **95%,** at least **96%,** at least **97%,** at least **98%,** or at least **99%** sequence identity, compared to the **VH** in the **(II-1);** and/or, having **VL** with at least **70%,** at least **80%,** at least **85%,** at least **90%,** at least **91%,** at least **92%,** at least **93%,** at least **94%,** at least **95%,** at least **96%,** at least **97%,** at least **98%,** or at least **99%** sequence identity, compared to the **VL** in the **(II-1);** or
(**II-3**), having **VH** in substitution, deletion, or addition of one or several amino acids or any combination thereof (for example, substitution, deletion, or addition of **1, 2, 3, 4, 5, 6, 7, 8, 9,** or **10** amino acids or any combination thereof), compared to the **VH** in the (**II-1**); and /or, having **VL** in substitution, deletion, or addition of one or several amino acids or any combination thereof (for example, substitution, deletion, or addition of **1, 2, 3, 4, 5, 6, 7, 8, 9,** or **10** amino acids or any combination thereof), compared to the **VL** in the **(II-1);** preferably, the substitution being conservative substitution.

In some embodiments, the **TSLP** antibody or the antigen-binding fragment thereof comprises:
(**III-1**) **VH** in a sequence set forth in the **SEQ ID NO:12** and **VL** in a sequence set forth in the **SEQ ID NO:13;**
(**III-2**) **VH** and **VL,** wherein, compared to the **VH** and the **VL** in the **(III-1),** the **VH** has at least **70%,** at least **80%,** at least **85%,** at least **90%,** at least **91%,** at least **92%,** at least **93%,** at least **94%,** at least **95%,** at least **96%,** at least **97%,** at least **98%,** or at least **99%** sequence identity; and/or the **VL** has at least **70%,** at least **80%,** at least **85%,** at least **90%,** at least **91%,** at least **92%,** at least **93%,** at least **94%,** at least **95%,** at least **96%,** at least **97%,** at least **98%,** or at least **99%** sequence identity; or
(**III-3**) **VH** and **VL,** wherein, compared to the **VH** and the **VL** in the **(III-1),** the **VH** has substitution, deletion, or addition of one or several amino acids or any combination thereof (for example, substitution, deletion, or addition of **1, 2, 3, 4, 5, 6, 7, 8, 9,** or **10** amino acids or any combination thereof); and/or, the **VL** has substitution, deletion, or addition of one or several amino acids or any combination thereof (for example, substitution, deletion, or addition of **1, 2, 3, 4, 5, 6, 7, 8, 9,** or **10** amino acids or any combination thereof). In some embodiments, the substitution is conservative substitution.

In some embodiments, the antibody or the antigen-binding fragment thereof is a chimeric antibody, a humanized antibody, or a fully human antibody.

In some embodiments, the **TSLP** antibody or the antigen-binding fragment thereof further comprises:
**(i-1)** a heavy chain constant region **(CH)** of a human immunoglobulin or a variant thereof; and/or
**(i-2)** a light chain constant region **(CL)** of a human immunoglobulin or a variant thereof,
wherein the variant has, compared to a wild-type sequence from which the variant is derived, at least **70%,** at least **80%,** at least **85%,** at least **90%,** at least **91%,** at least **92%,** at least **93%,** at least **94%,** at least **95%,** at least **96%,** at least **97%,** at least **98%,** or at least **99%** sequence identity; or the variant has, compared to the wild-type sequence from which the variant is derived, substitution, deletion, or addition of one or more amino acids or any combination thereof (for example, substitution, deletion, or addition of at most **50,** at most **45,** at most **40,** at most **35,** at most **30,** at most **25,** at most **20,** at most **15,** at most **10,** or at most **5** amino acids or any combination thereof; for example, substitution, deletion, or addition of **1, 2, 3, 4, 5, 6, 7, 8, 9,** or **10** amino acids or any combination thereof); and preferably, the substitution is conservative substitution.

In some embodiments, the heavy chain constant region is an **IgG** heavy chain constant region, such as an **IgG1**, **IgG2, IgG3,** or **IgG4** heavy chain constant region. In some embodiments, the antibody or the antigen-binding fragment thereof comprises a human **IgG1** heavy chain constant region. In some embodiments, the light chain constant region is a **κ** or **λ** light chain constant region. In some embodiments, the antibody or the antigen-binding fragment thereof comprises a human **κ** light chain constant region.

In some embodiments, the heavy chain constant region or the variant thereof comprises **CH** set forth in **SEQ ID NO:14** or a variant thereof, wherein the variant has, compared to the **SEQ ID NO:14,** conservative substitution of at most **20** amino acids (for example, conservative substitution of at most **20,** at most **15,** at most **10,** or at most **5** amino acids; for example, conservative substitution of **1, 2, 3, 4, 5, 6, 7** , **8, 9, or 10** amino acids), or has, compared to the **SEQ** ID **NO:14,** at least **70%,** at least **80%,** at least **85%,** at least **90%,** at least **91%,** at least **92%,** at least **93%,** at least **94%,** at least **95%,** at least **96%,** at least **97%,** at least **98%,** or at least **99%** sequence identity.

In some embodiments, the light chain constant region or the variant thereof comprises a light chain constant region **(CL)** set forth in **SEQ ID NO:15** or a variant thereof, wherein the variant has, compared to the **SEQ ID NO:15,** conservative substitution of at most **20** amino acids (for example, conservative substitution of at most **20,** at most **15,** at most **10,** or at most **5** amino acids; for example, conservative substitution of **1, 2, 3, 4, 5, 6, 7, 8, 9,** or **10** amino acids), or has, compared to the **SEQ ID NO:15,** at least **70%,** at least **80%,** at least **85%,** at least **90%,** at least **91%,** at least **92%,** at least **93%,** at least **94%,** at least **95%,** at least **96%,** at least **97%,** at least **98%,** or at least **99%** sequence identity.

In some embodiments, the antibody or the antigen-binding fragment thereof comprises a heavy chain constant region **(CH)** set forth in the **SEQ ID NO:14** and a light chain constant region **(CL)** set forth in the **SEQ ID NO:15.**

In some embodiments, the antibody comprises a heavy chain comprising the **VH** set forth in the **SEQ ID NO:12** and the **CH** set forth in the **SEQ ID NO:14,** and, a light chain comprising the **VL** set forth in the **SEQ ID NO:13** and the **CL** set forth in the **SEQ ID NO:15.**

In some embodiments, the **TSLP** antibody comprises a heavy-chain amino acid sequence set forth in **SEQ ID NO:16** and a light-chain amino acid sequence set forth in **SEQ ID NO:17.**

In some embodiments, the **TSLP** antibody or the antigen-binding fragment thereof is at a concentration of **100-350 mg/mL,** for example, **100-300 mg/mL, 100-250 mg/mL, 100-200 mg/mL,** or **150-200 mg/mL,** preferably **100-200 mg/mL,** more preferably **150-200 mg/mL,** still more preferably **135-165 mg/mL.**

Herein, the "**x±n**" describes a value range of a parameter, wherein the range includes a range where the amplitude of up and down changes of the eigenverte **x** is **n.** For example, the **TSLP** antibody or the antigen-binding fragment thereof is at a concentration of **150 mg/mL±15 mg/mL,** which means that the **TSLP** antibody or the antigen-binding fragment thereof is at a concentration of **135 mg/mL-165 mg/mL.**

In some embodiments, the adjuvant is selected from one or more of: a buffer agent, a stabilizer, a surfactant, and an osmotic pressure regulator.

In some embodiments, the buffer agent is selected from histidine/hisdidine hydrochloride buffer, citric acid/citrate buffer, and acetic acid/acetate buffer. In some embodiments, the buffer agent is at a **pH** of **5.5-6.5,** preferably **5.5-6.0,** preferably **5.7-6.3,** for example, **5.5, 5.7, 6.0,** or **6.3.** In some embodiments, the buffer agent is **10-30 mM** histidine/histidine hydrochloride buffer, more preferably **10.4-26.7 mM** histidine/histidine hydrochloride buffer, for example, **10 mM±2 mM** or **20** mM±2 mM histidine/histidine hydrochloride buffer.

In some embodiments, the stabilizer is selected from one or more of: sodium chloride, sucrose, trehalose, and an amino acid or a salt thereof (e.g., arginine or a salt thereof, lysine or a salt thereof, glycine, proline, or arginine-glutamic acid). In some embodiments, the stabilizer is a combination of sucrose and arginine hydrochloride. In some embodiments, each stabilizer is at a concentration of **15-200 mM,** for example, **50-200 mM, 60-200 mM, 50-120 mM,** or **60-120 mM.** In some embodiments, the sucrose is at a concentration of **100-200 mM,** for example, **108-132 mM;** and the arginine hydrochloride is at a concentration of **45-120 mM,** for example, **54-66 mM.** In some embodiments, the stabilizer is a combination of **108-132 mM** sucrose and **54-66 mM** arginine hydrochloride.

In some embodiments, the surfactant is a nonionic surfactant, such as polysorbate **20,** polysorbate **80,** or poloxamer. In some embodiments, the surfactant is poloxamer, preferably poloxamer **188.** In some embodiments, the surfactant is at a concentration of **0.01 w/v%-0.1 w/v%,** for example, **0.01 w/v%-0.03 w/v%, 0.01 w/v%-0.05 w/v%, 0.02 w/v%-0.03 w/v%, 0.02 w/v%-0.05 w/v%, 0.02 w/v%-0.1 w/v%, 0.03 w/v%-0.05 w/v%, 0.03 w/v%-0.1 w/v%,** or **0.05 w/v%-0.1 w/v%.** In some embodiments, the surfactant is poloxamer **188** at **0.01 w/v%-0.1 w/v%,** for example, poloxamer **188** at **0.01 w/v%-0.03 w/v%, 0.01 w/v%-0.05 w/v%, 0.02 w/v%-0.03 w/v%, 0.02 w/v%-0.05 w/v%, 0.02 w/v%-0.1 w/v%, 0.03 w/v%-0.05 w/v%, 0.03 w/v%-0.1 w/v%,** or **0.05 w/v%-0.1 w/v%,** preferably poloxamer **188** at **0.045-0.055 w/v%.** Unless otherwise specified, the **"w/v%"** herein refers to a massic volume concentration, representing the number of grams of a solute per **100** milliliters of liquid, and may also be expressed as **10 g/L.**

In some embodiments, the osmotic pressure regulator is sodium chloride. In some embodiments, the osmotic pressure regulator is at a concentration of **20-130 mM,** for example, **40-130 mM** or **60-130 mM;** and preferably, the osmotic pressure regulator is sodium chloride at **54-66 mM**.

In some embodiments, the pharmaceutical composition comprises the **TSLP** antibody at **135-165 mg/mL,** histidine/histidine hydrochloride buffer at **18-22 mM** at a **pH** of **5.7-6.3,** sucrose at **108-132 mM,** arginine hydrochloride at **54-66 mM,** and poloxamer **188 at 0.045-0.055 w/v%.**

In some embodiments, the pharmaceutical composition comprises the **TSLP** antibody at **150 mg/mL,** histidine/histidine hydrochloride buffer at **20 mM** at a **pH** of **6.0,** sucrose at **120 mM,** arginine hydrochloride at **60 mM,** and poloxamer **188** at **0.05 w/v%.**

In some embodiments, the pharmaceutical composition consists of the **TSLP** antibody at **150 g/L,** histidine at **1.09 g/L,** histidine hydrochloride at **2.73 g/L,** sucrose at **41.08 g/L,** arginine hydrochloride at **12.64 g/L,** and poloxamer **188** at **0.5 g/L.**

In some embodiments, the pharmaceutical composition is an injection, for example, a subcutaneous or intramuscular injection.

In some embodiments, the pharmaceutical composition is a solution.

In some embodiments, the pharmaceutical composition is a lyophilized powder.

In a second aspect, the present invention provides a preparation, comprising the pharmaceutical composition according to any one embodiment in the first aspect and a container accommodating the pharmaceutical composition.

In some embodiments, the container is a vial or a syringe.

In some embodiments, the preparation is a prefilled syringe or an autoinjector pen.

In a third aspect, the present invention provides a kit, comprising the pharmaceutical composition according to any one embodiment in the first aspect or the preparation according to any one embodiment in the second aspect, and a manual.

In another aspect, the present invention provides use of the pharmaceutical composition according to any one embodiment in the first aspect, the preparation according to any one embodiment in the second aspect, or the kit according to any one embodiment in the third aspect for the manufacture of a medicament for preventing and/or treating an allergic inflammation or an autoimmune disease.

In another aspect, the present invention provides use of the pharmaceutical composition according to any one embodiment in the first aspect, the preparation according to any one embodiment in the second aspect, or the kit according to any one embodiment in the third aspect for preventing and/or treating an allergic inflammation or an autoimmune disease.

In another aspect, the present invention provides a method for preventing and/or treating an allergic inflammation or an autoimmune disease, comprising administering to a subject in need thereof a prophylactically or therapeutically effective amount of the pharmaceutical composition according to any one embodiment in the first aspect, the preparation according to any one embodiment in the second aspect, or the kit according to any one embodiment in the third aspect.

In some embodiments, the allergic inflammation is selected from at least one of asthma, idiopathic pulmonary fibrosis, atopic dermatitis **(AD),** allergic conjunctivitis, allergic rhinitis **(AR)** Netherton syndrome, eosinophilic esophagitis **(EOE),** food allergy, allergic diarrhea, eosinophilic gastroenteritis, allergic bronchopulmonary aspergillosis **(ABPA),** allergic fungal sinusitis, rheumatoid arthritis, chronic obstructive pulmonary disease **(COPD),** systemic sclerosis, keloid, ulcerative colitis, chronic sinusitis **(CRS)** and nasal polyp, chronic eosinophilic pneumonia, eosinophilic bronchitis, Churg-Strauss syndrome, eosinophilia, eosinophilic granulomatosis with polyangiitis, inflammatory bowel disease, urticaria, systemic mastocytosis, skin mastocytosis, and recurrent idiopathic angioedema.

In some embodiments, the autoimmune disease is selected from: diabetes, myasthenia gravis, gastritis, pemphigus, primary biliary cirrhosis, multiple sclerosis, lupus, colitis, rheumatoid, psoriasis, and thyroid disease.

In some embodiments, the use or the method comprises administering the medicament in combination with one or more additional therapeutic agents, the additional therapeutic agents being selected from, but not limited to: immunosuppressant (e.g., corticosteroid, nonsteroidal glucocorticoid receptor agonist, leukotriene **D4** antagonist, leukotriene **B4** antagonist, **A2A** agonist, **A2B** antagonist, dopamine receptor agonist, pirfenidone, nintedanib, or **avB6** antagonist), bronchodilator (e.g., **β-2** adrenergic receptor agonist, muscarinic antagonist, short-acting **β2** receptor agonist, long-acting **β2** receptor agonist, short-acting anticholinergic drug, methylxanthine-based drug, and long-acting anticholinergic drug), an additional cytokine or cytokine receptor antagonist or antibody (e.g., **IL-13** antagonist, **IL-6** antagonist, **IL-1, IL-33, IL-25** or **TNF-α** antagonist, anti**-IgE** antibody, anti**-IL-13** antibody, anti**-IL31R** antibody, anti**-IL13** antibody, anti-endoglin antibody, anti**-IL1b** antibody, another anti**-TSLP** antibody, or anti-**hTSLPR** antibody), antibiotic, radiotherapy, leukotriene antagonist (e.g., montelukast, zafirlukast, or pranlukast), **PDE4** inhibitor (such as roflumilast or **xanthene),** antihistamine, or cough suppressant.

In some embodiments, the antibody or the antigen-binding fragment, the pharmaceutical composition, the preparation, or the kit antibody, and the additional therapeutic agents are administered simultaneously, separately, or sequentially.

### Definition of terms

Unless otherwise stated, the scientific and technological nouns used herein have the meanings as commonly understood by those skilled in the art. Further, operating procedures of molecular genetics, nucleic acid chemistry, cell culture, biochemistry, cytobiology, etc. used herein are routine procedures widely used in corresponding industries. Moreover, in order to better understand the present invention, the definitions and interpretations of related terms are provided below.

As used herein, the term "antibody" refers to an immunoglobulin molecule typically composed of two pairs of polypeptide chains (each pair having a light chain **(LC)** and a heavy chain **(HC)).** The antibody light chain can be classified as a **κ (kappa)** light chain and a **λ (lambda)** light chain. The heavy chain can be classified as **µ, δ, γ, α,** or **ε,** and isotypes of the antibody are defined as **IgM, IgD, IgG, IgA,** and **IgE** respectively. In the light chain and the heavy chain, the variable region and the constant region are linked by a **"J"** region having about **12** or more amino acids, and the heavy chain further comprises a **"D"** region having about **3** or more amino acids. Each heavy chain is composed of a heavy chain variable region **(VH)** and a heavy chain constant region **(CH).** The heavy chain constant region is composed of **3** domains **(CH1, CH2,** and **CH3).** Each light chain is composed of a light chain variable region **(VL)** and a light chain constant region **(CL).** The light chain constant region is composed of a domain **CL.** A constant domain does not directly participate in the binding of the antibody to the antigen, but shows a variety of effector functions, such as mediating the binding of immunoglobulins to host tissues or factors, including various cells of the immune system (like effector cells) and a first component **(C1q)** of a classical complement system. The **VH** and the **VL** regions may be further subclassified as hypervariable regions (termed complementarity determining regions **(CDRs)),** interspersed with conserved regions termed framework regions **(FRs).** Each of the **VH** and the **VL** is sequentially composed of: **FR1, CDR1, FR2, CDR2, FR3, CDR3,** and **FR4,** namely **3 CDRs** and 4 **FRs** arranged from the amino terminus to the carboxy terminus. The variable regions (the **VH** and the **VL)** of each heavy chain/light chain pair respectively form an antigen-binding site. The assignment of amino acids in various regions or domains may follow various numbering systems known in the art.

The term "complementary determining region" or **"CDR"** refers to an amino acid residue in an antibody variable region responsible for antigen binding. Each of the variable regions of the heavy and the light chains comprises three **CDRs** named **CDR1, CDR2,** and **CDR3.** Precise boundaries of these **CDRs** may be defined based on various numbering systems known in the art, for example, defined based on a **Kabat** numbering system **(**Kabat et al., Sequences of Proteins of Immunological Interest, 5th Ed. Public Health Service, National Institutes of Health, Bethesda, Md.,1991**), a Chothia** numbering system **(**Chothia & Lesk(1987) J. Mol. Biol. 196:901-917**;** Chothia et al. (1989) Nature 342:878-883**), an IMGT** numbering system **(**Lefranc et al., Dev. Comparat. Immunol. 27:55-77,2003**)** or an **AbM** numbering system **(**Martin ACR, Cheetham JC, Rees AR (1989) Modelling antibody hypervariable loops: A combined algorithm. Proc Natl Acad Sci USA 86:9268-9272**).** For a given antibody, those skilled in the art will readily identify **CDRs** defined by each numbering system. Further, a corresponding relationship between different numbering systems is well known to those skilled in the art (for example, see Lefranc et al., Dev. Comparat. Immunol. 27:55-77, 2003**).**

In the present invention, the **CDRs** comprised in the antibody or the antigen-binding fragment thereof can be determined based on various numbering systems known in the art, for example, determined based on the **Kabat,** the **Chothia,** the **IMGT,** or the **AbM** numbering system. In some embodiments, the **CDRs** comprised in the antibody or the antigen-binding fragment thereof are defined based on the **IMGT** or the **AbM** numbering system.

The term "framework region" or **"FR"** residues refer to those amino acid residues in an antibody variable region other than the **CDR** residues as defined above.

The term "antigen-binding fragment" of an antibody refers to a polypeptide of a fragment of an antibody, such as a polypeptide of a fragment of a full-length antibody, retains an ability to specifically bind a same antigen to which the full-length antibody binds, and/or competes with the full-length antibody for specific binding to the antigen, and is also referred to as an "antigen-binding moiety". See generally, Fundamental Immunology, Ch. 7 (Paul, W., ed., 2nd edition, Raven Press, N.Y. (1989**),** which is incorporated herein by reference in its entirety for all purposes. An antigen-binding fragment of an antibody may be produced by a recombinant **DNA** technique or by enzymatic or chemical cleavage of an intact antibody. Non-limiting examples of antigen-binding fragments include **Fab** fragment, **Fab'** fragment, **F(ab)'₂** fragment, **F(ab)'₃** fragment, **Fd, Fv, scFv, di-scFv, (scFv)₂, Fv** protein **("dsFv")** with a stable disulfide bond, single-domain antibody (sdAb, nano-antibody) and such polypeptides that contain at least a portion of antibodies sufficient to confer a specific antigen binding ability to the polypeptide. Engineered antibody variants are summarized in Holliger et al., 2005; Nat Biotechnol, 23: 1126-1136**.**

As used herein, the term "adjuvant" refers to a substance that is necessary in a process of preparing or blending a pharmaceutical preparation, other than an active ingredient. These substances are generally required to neither have physiological activity, nor affect the efficacy, content determination and stability of a medicament in a pharmaceutical preparation. The adjuvant is added mainly to facilitate preparing and clinically using the preparation. The adjuvant used in the pharmaceutical composition of the present invention is pharmaceutically acceptable and further compatible with an active ingredient.

As used herein, the term "pharmaceutically acceptable" refers to a carrier and/or an excipient that is pharmacologically and/or physiologically compatible with a subject and an active ingredient, is well known in the art (see, e.g., Remington's Pharmaceutical Sciences. edited by Gennaro AR, 19th ed. Pennsylvania: Mack Publishing Company,1995**),** and includes, but is not limited to, a **pH** regulator or a buffer agent, a stabilizer, a surfactant, an osmotic pressure regulator, a preservative, and the like. For example, the **pH** regulator or the buffer agent includes, but is not limited to, phosphate buffer, histidine/histidine hydrochloride buffer, citric acid/citrate buffer, and acetic acid/acetate buffer. The surfactant includes, but is not limited to, a cationic, anionic, or nonionic surfactant, such as poloxamer or sorbitol. The preservative includes, but is not limited to, various antibacterial and antifungal reagents, such as paraben, chlorobutanol, phenol, or sorbic acid. The osmotic pressure regulator includes, but is not limited to, sugar, **NaCl,** and an analog thereof. A stabilizer has the meaning commonly understood by those skilled in the art, can stabilize desired activity of an active ingredient in a medicament, and includes, but is not limited to, sodium glutamate, gelatin, **SPGA,** sugar (such as sorbitol, mannitol, starch, sucrose, lactose, glucan, or glucose), an amino acid or a salt thereof (such as glutamic acid, glycine, arginine or a salt thereof, lysine or a salt thereof, proline, arginine-glutamic acid), a protein (such as dried whey, albumin, or casein) or a degradation product thereof (such as lactalbumin hydrolysate), and the like.

The preparation may be prepared into an appropriate form based on a desired administration route, which may be a liquid suspension and/or a dried (lyophilized) powder, and other forms. Example administration forms include, e.g., a powder-filled, uncoated or enteric-coated capsule with a hard or soft shell, a dissolvable tablet, a caplet, an unencapsulated or encapsulated mini-tablet, a multiparticulate, a lozenge, a pastille, a granule, a microsphere, a nanoparticle, an injectable liquid preparation, a liquid pill, an oral solution, an oral suspension, a syrup, an elixir, a gel, a bulk emulsion, an aerosol, a gasoloid, a microemulsion or a nanoemulsion, a liposome, or a suppository. These preparations can be administered directly or can be prepared for addition to a subject's food or beverage, such as a dietary supplement.

As used herein, the term "administration" refers to physical introduction of the pharmaceutical composition or the preparation of the present invention into an individual using any of various methods and delivery systems known to those skilled in the art. Preferred administration route includes intravenous, intramuscular, subcutaneous, intraperitoneal, spinal or other parenteral administration route, such as by injection or infusion. The pharmaceutical composition and the preparation of the present invention are preferably administered parenterally, by injection, including but not limited to intravenous, intramuscular, intraarterial, intrathecal, intralymphatic, intralesional, intravesicular, intraorbital, intracardial, intradermal, intraperitoneal, transtracheal, subcutaneous, intraepithelial, intraarticular, infracapsule, subarachnoid, intraspinal, epidural, and intrasternal injection and infusion, and in vivo electroporation, or may be administered, for example, once, multiple times and/or over one or more extended periods.

As used herein, the "treatment" of an individual refers to any type of intervention or process performed on the individual, or administration of a medicament to the individual, for the purpose of reversing, ameliorating, suppressing, slowing, or preventing the onset, progression, development, severity, or recurrence of symptoms, complications or disorders associated with a disease.

As used herein, the term "effective amount" refers to an amount sufficient to achieve, or at least partially achieve, a desired effect. For example, a prophylactically effective amount for a disease refers to an amount sufficient to prevent, restrain, or delay the occurrence of the disease; and a therapeutically effective amount for a disease refers to an amount sufficient to cure or at least partially restrain the disease and complications thereof in a patient who has suffered from the disease. Determining such effective amounts is completely within capabilities of those skilled in the art. For example, an effective amount for therapeutic use will depend on a severity of a to-be-treated disease, an overall state of patient's own immune system, patient's general conditions such as age, body weight, and gender, a drug administration mode, other concurrently administered treatments, and the like.

As used herein, the term "subject" includes, but is not limited to, various animals, e.g., a mammal, such as a bovid, an equid, a caprid, a suilline, a canid, a felid, a leporidae, a rodent (e.g., mouse or rat), a non-human primate (e.g., macaque or cynomolgus monkey), or a human.

The pharmaceutical composition in the present invention is "stable" with the **TSLP** antibody or the antigen-binding fragment thereof being stable under typical processing, storage and/or treatment stressors, such as mechanical stress, thermal stress, and/or freeze-thaw stress. In general, the preparation remains stable after prolonged storage at a particular temperature, after a freeze-thaw cycle, and/or after stirring. In general, a "stable" protein shows smallest (or no) alteration, smallest (or no) degradation or aggregation, smallest (or no) breakage sign, and/or chemical modification (e.g., oxidation, reduction, deamidation), etc. in secondary and tertiary structures, and retains the integrity of primary structure of the protein, so that it retains its physicochemical stability and bioactivity during storage, even when being subjected to a stressor. In some embodiments, compared to initial mass before storage, a freeze-thaw cycle or a stirring stress test, mass of the antibody or the antigen-binding fragment thereof is changed by no more than **60%,** preferably no more than **50%,** no more than **20%,** no more than **15%,** no more than **10%,** no more than **5%,** no more than **2%,** or no more than **1%** of total mass of the **TSLP** antibody.

### Detailed Description

Technical solutions in embodiments of the present invention will be clearly and completely described below. Apparently, the embodiments described below are merely a part, instead of all, of the embodiments of the present invention. The following description of at least one example embodiment is in fact merely illustrative, and definitely does not serve as any limitation to the present invention and its application or use. All other embodiments obtained by those of ordinary skills in the art based on the embodiments of the present disclosure without making creative work are encompassed within the scope of protection of the present disclosure.

### Sequence information

Information on sequences involved in the present invention is described in the following table.

| **SEQ ID NO:** | Description | Sequence information |
|---|---|---|
| **1** | **IMGT 43B1-H2L2 CDR-H1** | **GFTFRSYD** |
| **2** | **IMGT 43B1-H2L2 CDR-H2** | **IWYDGSNE** |
| **3** | **IMGT 43B1-H2L2 CDR-H3** | **ARSPLWYGEPDDAFDI** |
| **4** | **IMGT 43B1-H2L2 CDR-L1** | **QSVSSN** |
| **5** | **IMGT 43B1-H2L2 CDR-L2** | **GAS** |
| **6** | **IMGT/AbM 43B1-H2L2 CDR-L3** | **QHYNNWPLT** |
| **7** | **AbM 43B1-H2L2 CDR-H1** | **GFTFRSYDMH** |
| **8** | **AbM 43B1-H2L2 CDR-H2** | **VIWYDGSNEN** |
| **9** | **AbM 43B1-H2L2 CDR-H3** | **SPLWYGEPDDAFDI** |
| **10** | **AbM 43B1-H2L2 CDR-L1** | **RASQSVSSNLA** |
| **11** | **AbM 43B1-H2L2 CDR-L2** | **GASTRAT** |
| **12** | Antibody **43B1-H2L2** heavy chain variable region | QMQLVESGGGAVQPGGSLRLSCAASG FTFRSYDMHWVRQAPGKGLEWVAVI WYDGSNENYADSVKGRFTTSRDNSKN TLFLQMNSLRAEDTAVYYCARSPLWY GEPDDAFDIWGQGTLVTVSS |
| **13** | Light chain variable region of antibody **43B1-H2L2** | EIVMTQSPATLSVSPGERATLSCRASQS VSSNLAWYQQKPGQAPRLLISGASTRA TGIPARFSGSGSGTDFTLTISSLQSEDF AVYYCQHYNNWPLTFGGGTKVEIK |
| **14** | Modified human **IgG1** heavy chain constant region | ASTKGPSVFPLAPSSKSTSGGTAALGC LVKDYFPEPVTVSWNSGALTSGVHTFP AVLQSSGLYSLSSVVTVPSSSLGTQTYI CNVNHKPSNTKVDKKVEPKSCDKTHT CPPCPAPEAAGAPSVFLFPPKPKDTLM ISRTPEVTCVVVDVSHEDPEVKFNWYV DGVEVHNAKTKPREEQYNSTYRVVSV LTVLHQDWLNGKEYKCKVSNKALPA |
| | | PIEKTISKAKGQPREPQVYTLPPSRDEL TKNQVSLTCLVKGFYPSDIAVEWESNG QPENNYKTTPPVLDSDGSFFLYSKLTV DKSRWQQGNVFSCSVMHEALHAHYT QKSLSLSPGK |
| **15** | Human **κ** light chain constant region | |
| **16** | Heavy-chain amino acid sequence of antibody **43B1-H2L2** | QMQLVESGGGAVQPGGSLRLSCAASG FTFRSYDMHWVRQAPGKGLEWVAVI WYDGSNENYADSVKGRFTTSRDNSKN TLFLQMNSLRAEDTAVYYCARSPLWY GEPDDAFDIWGQGTLVTVSSASTKGPS VFPLAPSSKSTSGGTAALGCLVKDYFP EPVTVSWNSGALTSGVHTFPAVLQSSG LYSLSSVVTVPSSSLGTQTYICNVNHKP SNTKVDKKVEPKSCDKTHTCPPCPAPE AAGAPSVFLFPPKPKDTLMISRTPEVT CVVVDVSHEDPEVKFNWYVDGVEVH NAKTKPREEQYNSTYRVVSVLTVLHQ DWLNGKEYKCKVSNKALPAPIEKTISK AKGQPREPQVYTLPPSRDELTKNQVSL TCLVKGFYPSDIAVEWESNGQPENNY KTTPPVLDSDGSFFLYSKLTVDKSRWQ QGNVFSCSVMHEALHAHYTQKSLSLS PGK |
| **17** | Light chain amino acid sequence of antibody **43B1-H2L2** | EIVMTQSPATLSVSPGERATLSCRASQS VSSNLAWYQQKPGQAPRLLISGASTRA TGIPARFSGSGSGTDFTLTISSLQSEDF AVYYCQHYNNWPLTFGGGTKVEIKRT VAAPSVFIFPPSDEQLKSGTASVVCLLN NFYPREAKVQWKVDNALQSGNSQESV TEQDSKDSTYSLSSTLTLSKADYEKHK VYACEVTHQGLSSPVTKSFNRGEC |

In the embodiments, the detection methods in the 2020 edition of the General Rules of the Chinese Pharmacopoeia may be referred to for detection of **SEC-HPLC, IEC-HPLC,** reduced **CE-SDS,** non-reduced **CE-SDS,** osmotic pressure, isoelectric point, color, clarity, insoluble particles, and visible foreign matters. Viscosity is detected using an **m-VROC** microfluidic rheometer. Relative binding activity is detected by enzyme-linked immunosorbent assay (ELISA). Bioactivity is detected by proliferation inhibition.

### Experimental Example 1

**43B1-H2L2** antibody **(TSLP** antibody with a heavy chain set forth in **SEQ ID NO:16** with a light chain set forth in **SEQ ID NO:17 prepared** with reference to WO2021115240A1) with a molecular weight of about **145.4 kDa** with a **pI** value of **7.92.** The **43B1-H2L2** antibody was prepared into a preparation in Table **1,** and placed at **40°C** for **28 d,** to investigate stability of the **43B1-H2L2** antibody in acetic acid/sodium acetate buffer, citric acid/sodium citrate buffer, and histidine/histodine hydrochloride buffer, with the results as shown in Table **2.**

**Table 1 Preparation**

| Serial No. | Buffer system | **pH** | Buffer system concentration | Protein concentration |
|---|---|---|---|---|
| Preparation **1-1** | Acetic acid/sodium acetate | **5.5** | **10 mM** | **5.0 mg/ml** |
| Preparation **1-2** | Citric acid/sodium citrate | **5.5** | **10 mM** | **5.0 mg/ml** |
| Preparation **1-3** | Histidine/histidine hydrochloride | **5.5** | **10 mM** | **5.0 mg/ml** |

**Table 2 Experimental results**

| Preparation | Sampling point | Non-reduced **CE-SDS (%)** Main Peak | Reduced **CE-SDS (%) HC+LC** | **IEC-HPLC (%)** Main Peak | **SEC-HPLC (%)** Monomer |
|---|---|---|---|---|---|
| Preparation **1-1** | **T0** | **96.4** | **98.8** | **60.7** | **99.8** |
| | **40°C 28 d** | **92.0** | **94.2** | **48.4** | **94.5** |
| Preparation **1-2** | **T0** | **96.1** | **98.8** | **60.3** | **99.8** |
| | **40°C 28 d** | **94.5** | **94.4** | **45.3** | **96.4** |
| Preparation **1-3** | **T0** | **95.9** | **98.6** | **61.4** | **99.8** |
| | **40°C 28 d** | **94.7** | **94.7** | **49.5** | **96.7** |

| | | | | | |
|---|---|---|---|---|---|
| Note: **40°C 28 d** refers to placement at **40°C** for **28** days, similarly below. | | | | | |

The **43B1-H2L2** antibody is relatively stable in the histidine/hisdidine hydrochloride buffer system, and is better than in the acetic acid buffer system and the citric acid buffer system.

### Experimental Example 2

In this experiment, **10 mM** histidine/histidine hydrochloride buffer system was used to regulate the **pH to 5.5-6.0,** and prepare **43B1-H2L2** antibody into a preparation with a protein concentration of about **185 mg/ml** with the ingredients as shown in Table **3** below. The experiment was carried out under conditions of repeated freeze-thaw at **-20°C 4** times or placement at **40°C** for **28 d,** to investigate the influence of the combination of arginine hydrochloride and arginine-glutamic acid respectively with polysorbate **20** on the protein stability and viscosity. The detection method of each parameter is same as above.

**Table 3 Preparation**

| Preparation | Buffer/protein concentration | Polysorbate 20 **(w/v%)** | Arginine-glutamic acid | Arginine hydrochloride |
|---|---|---|---|---|
| Preparation **2-1** | **10 mM** histidine/histidine hydrochloride buffer system; protein concentration about **185 mg/ml** | **0.02%** | / | / |
| Preparation **2-2** | | **0.02%** | Each **120 mM** | / |
| Preparation **2-3** | | **0.02%** | / | **200 mM** |

**Table 4 Experimental results**

| Preparation | Sampling point | **IEC-HPLC (%)** Main Peak | **SEC-HPLC (%)** Monomer | Non-reduced **CE-SDS (%)** Main Peak | Reduced **CE-SDS (%) HC+LC** |
|---|---|---|---|---|---|
| Preparation **2-1** | **T0** | **57.8** | **99.0** | **96.7** | **97.8** |
| | **FT4X** | **57.7** | **96.1** | **96.0** | **98.0** |
| | **40°C 28 d** | **48.4** | **95.2** | **93.8** | **94.2** |
| Preparation **2-2** | **T0** | **57.9** | **99.1** | **97.0** | **98.2** |
| | **FT4X** | **57.0** | **99.1** | **96.6** | **98.4** |
| | **40°C 28 d** | **48.3** | **97.1** | **93.6** | **95.0** |
| Preparation **2-3** | **T0** | **57.9** | **99.0** | **97.0** | **98.3** |
| | **FT4X** | **57.9** | **99.0** | **96.8** | **98.1** |
| | **40°C 28 d** | **50.7** | **96.9** | **93.8** | **94.6** |

| | | | | | |
|---|---|---|---|---|---|
| Note: **FT4X** means repeated freeze-thaw at **-20°C 4** times. | | | | | |

Viscosity values of preparations **2-1, 2-2,** and **2-3** measured on day **0** are **33.5 cp, 13.6 cp,** and **10.8 cp,** respectively, showing that in the histidine/histidine hydrochloride system, arginine-glutamic acid and arginine hydrochloride can reduce the viscosity of the antibody preparation, and arginine hydrochloride has a better viscosity reducing effect.

In the above histidine/histidine hydrochloride system, arginine-glutamic acid and arginine hydrochloride can maintain the freeze-thaw stability and high-temperature stability of the **43B1-H2L2** antibody.

### Experimental Example 3

In this experiment, **10 mM** histidine/histidine hydrochloride buffer system was used to regulate the **pH** to about **6.0,** and prepare **43B1-H2L2** antibody into a preparation with a protein concentration of **150 mg/ml** with the ingredients as shown in Table **5** below. The experiment was carried out under conditions of repeated freeze-thaw at **-70°C 4** times or placement at **40°C** for **27 d,** to investigate the influence of the combination of polysorbate **80** with different adjuvants on the protein stability and viscosity. The detection method of each parameter is same as above.

**Table 5 Preparation**

| Preparation | Buffer/protein concentration | Lysine hydrochl oride | Arginine hydrochl oride | Sodium chloride | Glycine | Proline | Sucrose |
|---|---|---|---|---|---|---|---|
| Preparation **3-1** | **10 mM** histidine/hisdidine hydrochloride buffer system; Protein concentration **150 mg/ml;** Polysorbate 80 **0.02% (w/v%)** | / | **60 mM** | **60 mM** | / | / | / |
| Preparation **3-2** | | **15 mM** | **60 mM** | **60 mM** | / | / | / |
| Preparation **3-3** | | / | **60 mM** | / | / | **120 mM** | / |
| Preparation **3-4** | | / | **60 mM** | / | **120 mM** | / | / |
| Preparation **3-5** | | / | **60 mM** | / | / | / | **120 mM** |
| Preparation **3-6** | | / | / | / | / | / | / |

**Table 6 Experimental results**

| Preparation | Sampling point | **SEC-HPLC (%)** Monomer | Non-reduced **CE-SDS (%)** Main Peak | Reduced **CE-SDS (%) HC+LC** |
|---|---|---|---|---|
| Preparation **3-1** | **T0** | **98.4** | **96.1** | **98.8** |
| | **40°C 27 d** | **96.3** | **93.7** | **97.1** |
| Preparation **3-2** | **T0** | **98.4** | **96.2** | **98.5** |
| | **40°C 27 d** | **96.4** | **93.8** | **97.0** |
| Preparation **3-3** | **T0** | **98.4** | **96.3** | **98.5** |
| | **40°C 27 d** | **96.7** | **94.1** | **96.8** |
| Preparation **3-4** | **T0** | **98.4** | **96.0** | **98.5** |
| | **40°C 27 d** | **96.3** | **93.4** | **96.6** |
| Preparation **3-5** | **T0** | **98.4** | **96.0** | **98.6** |
| | **40°C 27 d** | **96.5** | **94.2** | **96.4** |

| | | | | |
|---|---|---|---|---|
| Note: **40°C 27 d** refers to placement at **40°C** for **27** days. | | | | |

Viscosity values of preparations **3-1, 3-2, 3-3, 3-4, 3-5,** and **3-6** measured on day **0** are **7.2 cp, 7.0 cp, 7.6 cp, 7.8 cp, 8.5 cp, and 17.3 cp** respectively, showing that in the above preparations, the combination of arginine hydrochloride with various adjuvants can reduce the viscosity of the antibody preparations.

After repeated freeze-thaw at **-70°C 4** times, each preparation in Table **5** remains clear appearance without visible protein particles, without obvious difference from purity indicators of main peaks of **IEC-HPLC** and monomers of **SEC-HPLC** on day **0,** without obvious difference among the preparations, and with freeze-thaw stability satisfying the requirements.

After placement at **40°C** for **27** days, each preparation in Table **5** has no obviously different stability, without obvious difference from main peak indicators of **IEC-HPLC** on day **0,** without obvious difference from purity indicators of monomers of **SEC-HPLC,** non-reduced **CE-SDS,** and reduced **CE-SDS** on day **0,** without obvious difference among the preparations, and with stability satisfying the requirements.

### Experimental Example 4

In this experiment, **20 mM** histidine/histidine hydrochloride buffer system was used to regulate the **pH** to about **6.0,** prepare **43B1-H2L2** antibody into a preparation with a protein concentration of **150 mg/ml** with the ingredients as shown in Table **7** below, and investigate the influence of polysorbate **80,** polysorbate **20,** and poloxamer **188** on the protein stability and viscosity. The detection method of each parameter is same as above.

**Table 7 Preparation**

| Preparation | Buffer/protein concentration | Arginine hydrochlo ride | Polysorbate **20 (w/v%)** | Polysorbate **80 (w/v%)** | Polosham **188 (w/v%)** | Sodium chloride |
|---|---|---|---|---|---|---|
| Preparation **4-1** | **20 mM** histidine/histidine hydrochloride buffer system; protein concentration **150 mg/ml** | / | / | / | / | / |
| Preparation **4-2** | | **60 mM** | **0.02** | / | / | **60 mM** |
| Preparation **4-3** | | **60 mM** | / | **0.02** | / | **60 mM** |
| Preparation **4-4** | | **60 mM** | / | / | **0.05** | **60 mM** |

**Table 8 Experimental results**

| Preparation | Sampling point | **IEC-HPLC (%)** Main Peak | **SEC-HPLC (%)** Monomer | Non-reduced **CE-SDS** (%) Main Peak | Reduced **CE-SDS (%) HC+LC** |
|---|---|---|---|---|---|
| Preparation **4-2** | **T0** | **69.3** | **98.8** | **95.9** | **97.2** |
| | **40°C 28 d** | **58.0** | **97.3** | **92.9** | **94.8** |
| Preparation **4-3** | **T0** | **69.2** | **98.8** | **95.9** | **97.2** |
| | **40°C 28 d** | **57.2** | **97.1** | **93.1** | **94.8** |
| Preparation **4-4** | **T0** | **69.2** | **98.8** | **96.1** | **97.0** |
| | **40°C 28 d** | **58.2** | **97.3** | **93.3** | **94.9** |

Viscosity values of preparations **4-1, 4-2, 4-3,** and **4-4** measured on day **0** are **10.1 cp, 6.1 cp, 6.5 cp,** and **6.5** cp respectively, showing that in the above histidine/hisdidine hydrochloride system, arginine hydrochloride at **≥60 mM** can satisfy the requirements for viscosity of the preparations.

On day 0, the preparations **4-2, 4-3,** and **4-4** have better appearance than the preparation **4-1.** After placement at **40°C** for **28 d,** the preparation **4-4** is observed to have better appearance stability than the preparations **4-2** and **4-3,** showing that the preparation prepared from poloxamer **188** has better stability in appearance indicators than polysorbate **20** and polysorbate **80.**

The results in Table **8** show that the preparations **4-2, 4-3,** and **4-4** do not have obviously different stability, all satisfying the quality requirements.

### Experimental Example 5

In this experiment, **20 mM** histidine/histidine hydrochloride buffer system was used to regulate the **pH** to about **6.0,** and prepare **43B1-H2L2** antibody into a preparation with a protein concentration of **150 mg/ml** with the ingredients as shown in Table **9** below. The resulting preparation was placed at **40°C** for **28 d,** to investigate the influence of the combination of sodium chloride, glycine, and sucrose respectively with arginine hydrochloride and poloxamer **188** on the protein stability and viscosity. The detection method of each parameter is same as above.

**Table 9 Preparation**

| Preparation | Buffer/protein concentration | Arginine hydrochloride | Poloxamer **188 (w/v%)** | Sodium chloride | Glycine | Sucrose |
|---|---|---|---|---|---|---|
| Preparation **5-1** | **20 mM** histidine/histidine hydrochloride buffer system; protein concentration **150 mg/ml** | **60 mM** | **0.05** | / | **120 mM** | / |
| Preparation **5-2** | | **60 mM** | **0.05** | / | / | **120 mM** |
| Preparation **5-3** | | **60 mM** | **0.05** | **60 mM** | / | / |

**Table 10 Experimental results**

| Preparation | Sampling point | **IEC-HPLC (%)** Main Peak | **SEC-HPLC (%)** Monomer | Non-reduced **CE-SDS (%)** Main Peak | Reduced **CE-SDS (%) HC+LC** |
|---|---|---|---|---|---|
| Preparation **5-1** | **T0** | **66.9** | **99.2** | **95.6** | **97.0** |
| | **40°C 28 d** | **55.7** | **97.3** | **93.5** | **93.6** |
| Preparation **5-2** | **T0** | **67.1** | **99.1** | **95.7** | **97.3** |
| | **40°C 28 d** | **56.6** | **97.2** | **93.7** | **93.9** |
| Preparation **5-3** | **T0** | **67.0** | **99.1** | **95.7** | **97.3** |
| | **40°C 28 d** | **57.0** | **97.1** | **93.4** | **93.6** |

In this experiment, each of the three groups of preparations has a viscosity of lower than **7.0 cp.** After placement at **40°C** for **28 d,** each of the three groups of preparations does not have obvious appearance changes, satisfying the requirements; and does not have obviously different stability in purity indicators.

### Experimental Example 6

In this experiment, **20 mM** histidine/histidine hydrochloride buffer system was used to select concentrations of poloxamer **188,** regulate the **pH** to about **6.0,** prepare **43B1-H2L2** antibody into a preparation with a protein concentration of **150 mg/ml** with the ingredients as shown in Table **11** below, and investigate the influence of different concentrations of poloxamer **188** on the protein stability. The detection method of each parameter is same as above.

**Table 11 Preparation**

| Preparation | Buffer/protein concentration | Arginine hydrochloride | Poloxamer **188 (w/v%)** | Sucrose |
|---|---|---|---|---|
| Preparation **6-1** | **20 mM** histidine/histidine hydrochloride buffer system; protein concentration **150 mg/ml** | **60 mM** | **0.01** | **120 mM** |
| Preparation **6-2** | | **60 mM** | **0.03** | **120 mM** |
| Preparation **6-3** | | **60 mM** | **0.05** | **120 mM** |
| Preparation **6-4** | | **60 mM** | **0.10** | **120 mM** |

**Table 12 Experimental results**

| Preparation | Sampling point | **IEC-HPLC (%)** Main Peak | **SEC-HPLC (%)** Monomer | Non-reduced **CE-SDS (%)** Main Peak | Reduced **CE-SDS (%) HC+LC** |
|---|---|---|---|---|---|
| Preparation **6-1** | **T0** | **66.1** | **99.2** | **95.1** | **97.4** |
| | **AG4h** | **66.3** | **99.2** | **95.0** | **97.1** |
| | **FT4X** | **66.2** | **99.2** | **95.1** | **97.2** |
| | **40°C 28 d** | **56.9** | **97.1** | **92.7** | **94.4** |

| | | | | | |
|---|---|---|---|---|---|
| Note: **AG4h** means oscillation for **4** h. | | | | | |

Preparation **6-1** does not have obvious appearance changes under investigation conditions of oscillation, freeze-thaw, and **40°C,** satisfying the requirements; does not have obvious changes of purity indicators under oscillation and freeze-thaw conditions; and has slightly decreased purity indicators at **40°C,** but all satisfy the requirements.

Preparations **6-2, 6-3,** and **6-4** are not obviously different from the preparation **6-1** in any of the investigation indicators under investigation conditions of oscillation, freeze-thaw, and **40°C.**

The poloxamer **188** at a concentration of **0.01%-0.10 w/v%** has consistent influence on the preparation stability without obvious difference.

### Experimental Example 7

This experimental formula comprising an antibody, histidine/histidine hydrochloride, arginine hydrochloride, sucrose, and poloxamer **188** is intended to investigate the stability of the preparation formula with a **pH** value of **5.7** and **6.3,** when the protein concentration, buffer salt, and adjuvant concentration change.

**Table 13 Preparation**

| Preparation | **pH** | Protein concentration **mg/ml** | Histidine/histidine hydrochloride **(mM)** | Poloxamer **188 (w/v%)** | Arginine hydrochloride **(mM)** | Sucrose **(mM)** |
|---|---|---|---|---|---|---|
| Preparation **7-1** | **6.0** | **150** | **20** | **0.05** | **60** | **120** |
| Preparation **7-2** | **6.0** | **150** | **10.4** | **0.05** | **60** | **120** |
| Preparation **7-3** | **6.0** | **150** | **26.7** | **0.05** | **60** | **120** |
| Preparation **7-4** | **5.7** | **150** | **20** | **0.05** | **60** | **120** |
| Preparation **7-5** | **6.3** | **150** | **20** | **0.05** | **60** | **120** |
| Preparation **7-6** | **6.0** | **150** | **20** | **0.045** | **54** | **108** |
| Preparation **7-7** | **6.0** | **150** | **20** | **0.055** | **66** | **132** |
| Preparation **7-8** | **6.0** | **135** | **20** | **0.05** | **60** | **120** |
| Preparation **7-9** | **6.0** | **165** | **20** | **0.05** | **60** | **120** |

**Table 14 Experimental results**

| Preparation | Sampling point | Appearance | **IEC-HPLC (%)** Main Peak | **SEC-HPLC (%)** Monomer | Non-reduced **CE-SDS (%)** Main Peak | Reduced **CE-SDS (%) HC+LC** |
|---|---|---|---|---|---|---|
| Preparation **7-1** | **T0** | Clear without visible protein particles | **63.7** | **99.3** | **94.8** | **97.4** |
| | **AG4h** | | **64.2** | **99.2** | **95.0** | **97.5** |
| | **FT4X** | | **63.5** | **99.2** | **94.8** | **97.6** |
| | **40°C 28 d** | | **55.8** | **97.2** | **93.2** | **95.3** |
| | **25°C 90 d** | | **62.4** | **98.1** | **94.4** | **97.1** |

Under the investigation conditions of oscillation, freeze-thaw, **25°C,** and **40°C,** the appearance of the preparation **7-1** satisfies the requirements, each of the purity indicators shows that the preparation is relatively stable, and the relative binding activity of the protein is changed by less than **15%.**

Under the investigation conditions of oscillation, freeze-thaw, **25°C,** and **40°C,** the other **8** groups of preparations (preparations **7-2-7-9)** in this experiment are not obviously different from the preparation **7-1** in each inspection indicator, all satisfying the requirements.

### Experimental conclusions

**1) 43B1-H2L2** antibody at a concentration of **150 mg/ml, 20 mM** histidine/histidine hydrochloride buffer system at **pH 6.0, 60 mM** arginine hydrochloride, **120 mM** sucrose, and poloxamer **188** at **0.05 w/v%** can satisfy the requirements for stability of the preparation;
**2)** based on **1)**, the preparation stability is not obviously different when the **pH** value is **5.7-6.3** with other conditions remaining unchanged;
**3)** based on **1),** histidine/histidine hydrochloride at a concentration of **10.4 mM-26.7 mM** has no obvious influence on the preparation stability with other conditions remaining unchanged;
**4)** based on **1),** the preparation stability is not obviously different when concentrations of the adjuvant arginine hydrochloride, sucrose, and poloxamer **188** fluctuate up and down within a range of **10%** with other conditions remaining unchanged; and
**5)** based on **1),** when the protein concentration fluctuates up and down within a range **of 10%** with other conditions remaining unchanged, the viscosity is affected, but satisfies the requirements, and other stability indicators are not obviously different.

### Experimental Example 8

Preparing **43B1-H2L2** antibody injection: **43B1-H2L2** antibody at **150 g/L,** histidine at **1.09 g/L,** histidine hydrochloride at **2.73 g/L,** sucrose at **41.08 g/L,** arginine hydrochloride at **12.64 g/L,** and poloxamer **188** at **0.5 g/L.** The injection was encapsulated in an injection vial. The injection vial was placed upside-up and upside-down respectively at **5°C±3°C**, to investigate the long-term stability.

### Experimental results

Compared to the **0-month** data, the **12-month** investigation data shows that none of the color, visible foreign matter, clarity, insoluble particles, **pH,** and osmotic pressure molar concentration is obviously changed, all satisfying the requirements;
compared to the **0**-month data, the **12**-month data of the protein content is changed by less than **4%;**
in **0-12** months, the monomer content of **SEC-HPLC** is not obviously changed, and is not less than **99.0%;** the main peaks of **IEC-HPLC** are not obviously changed, and are not less than **65%;** the content of heavy and light chains (**HC+LC**) of reduced CE-SDS is not significantly changed, and is not less than **97%;** the main peaks of non-reduced **CE-SDS** are not obviously changed, and are not less than **95%;** and
comparison between the **12**-month data and the **0**-month data shows that the relative binding activity is changed by no more than **10%,** and the bioactivity is changed by no more than **10%,** all satisfying the requirements.

In summary, after prolonged placement of the **43B1-H2L2** antibody injection at **5°C±3°C** for **12** months, the sample is stable without obvious changes of the investigation items.

In addition to those described herein, various modifications of the present invention will be apparent to those skilled in the art based on the foregoing description. Such modifications are also intended to be encompassed within the scope of the appended claims. The references cited in the present application (including all patents, patent applications, journal articles, books, and any other publications) are incorporated herein by reference in their entirety.

## Claims

1. A pharmaceutical composition containing a **TSLP** (thymic stromal lymphopoietin) antibody or an antigen-binding fragment thereof, comprising **50-400 mg/mL** of the **TSLP** antibody or the antigen-binding fragment thereof, and an adjuvant.

2. The pharmaceutical composition according to claim **1,** wherein the **TSLP** antibody comprises a heavy-chain amino acid sequence set forth in **SEQ ID NO:16** and a light-chain amino acid sequence set forth in **SEQ ID NO:17.**

3. The pharmaceutical composition according to claim **1** or **2,** wherein the **TSLP** antibody or the antigen-binding fragment thereof is at a concentration of **100-350 mg/mL,** for example, **100-300 mg/mL, 100-250 mg/mL, 100-200 mg/mL,** or **150-200 mg/mL,** preferably **100-200 mg/mL,** more preferably **150-200 mg/mL,** and still more preferably **135-165 mg/mL.**

4. The pharmaceutical composition according to any one of claims 1-3, wherein the adjuvant is selected from one or more of: a buffer agent, a stabilizer, a surfactant, and an osmotic pressure regulator;
preferably, the buffer agent is selected from histidine/histidine hydrochloride buffer, citric acid/citrate buffer, and acetic acid/acetate buffer; preferably, the buffer agent is at a **pH** of **5.5-6.5,** preferably **5.5-6.0,** preferably **5.7-6.3,** for example, **5.5, 5.7, 6.0,** or **6.3;** and preferably, the buffer agent is **10-30 mM** histidine/histidine hydrochloride buffer, more preferably **10.4-26.7 mM** histidine/histidine hydrochloride buffer, for example, **10 mM±2 mM** or **20 mM±2 mM** histidine/histidine hydrochloride buffer;
preferably, the stabilizer is selected from one or more of: sodium chloride, sucrose, trehalose, and an amino acid or a salt thereof (e.g., arginine or a salt thereof, lysine or a salt thereof, glycine, proline, arginine-glutamic acid); preferably, the stabilizer is a combination of sucrose and arginine hydrochloride; each stabilizer is at a concentration of **15-200 mM,** for example, **50-200 mM, 60-200 mM, 50-120 mM,** or **60-120 mM;** preferably, the sucrose is at a concentration of **100-200 mM,** for example, **108-132 mM;** the arginine hydrochloride is at a concentration of **45-120 mM,** for example, **54-66 mM;** preferably, the stabilizer is a combination of **108-132 mM** sucrose and **54-66 mM** arginine hydrochloride;
preferably, the surfactant is a nonionic surfactant, such as polysorbate **20,** polysorbate **80,** or poloxamer; preferably, the surfactant is poloxamer, preferably poloxamer **188;** the surfactant is at a concentration of **0.01 w/v%-0.1 w/v%, for example, 0.01 w/v%-0.03 w/v%, 0.01 w/v%-0.05 w/v%, 0.02 w/v%-0.03 w/v%, 0.02 w/v%-0.05 w/v%, 0.02 w/v%-0.1 w/v%, 0.03 w/v%-0.05 w/v%, 0.03 w/v%-0.1 w/v%,** or **0.05 w/v%-0.1 w/v%;** preferably, the surfactant is poloxamer **188** at **0.01 w/v%-0.1 w/v%,** for example, **0.01 w/v%-0.03 w/v%, 0.01 w/v%-0.05 w/v%, 0.02 w/v%-0.03 w/v%, 0.02 w/v%-0.05 w/v%, 0.02 w/v%-0.1 w/v%, 0.03 w/v%-0.05 w/v%,** or **0.03 w/v%-0.1 w/v%,** or poloxamer **188** at **0.05 w/v%-0.1 w/v%,** preferably poloxamer **188** at **0.045-0.055 w/v%;**
preferably, the osmotic pressure regulator is sodium chloride; preferably, the osmotic pressure regulator is at a concentration of **20-130 mM,** for example, **40-130 mM** or **60-130 mM;** and preferably, the osmotic pressure regulator is sodium chloride at **54-66 mM.**

5. The pharmaceutical composition according to any one of claims **1-4,** wherein the pharmaceutical composition comprises the **TSLP** antibody at **135-165 mg/mL,** histidine/histidine hydrochloride buffer at **18-22 mM** at a **pH** of **5.7-6.3,** sucrose at **108-132 mM,** arginine hydrochloride at **54-66 mM,** and poloxamer **188** at **0.045-0.055 w/v%;**
preferably, the pharmaceutical composition comprises the **TSLP** antibody at **150 mg/mL,** histidine/histidine hydrochloride buffer at **20 mM** at a **pH** of **6.0,** sucrose at **120 mM,** arginine hydrochloride at **60 mM,** and poloxamer **188** at **0.05 w/v%;** and
preferably, the pharmaceutical composition consists of the **TSLP** antibody at **150** g/L, histidine at **1.09 g/L,** histidine hydrochloride at **2.73 g/L,** sucrose at **41.08 g/L,** arginine hydrochloride at **12.64 g/L,** and poloxamer **188** at **0.5 g/L.**

6. The pharmaceutical composition according to any one of claims **1-5,** wherein the pharmaceutical composition is an injection, for example, a subcutaneous or intramuscular injection;
preferably, the pharmaceutical composition is a liquid solution; and
preferably, the pharmaceutical composition is a lyophilized powder.

7. A preparation, comprising the pharmaceutical composition according to any one of claims 1-6 and a container accommodating the pharmaceutical composition; wherein
preferably, the container is a vial or a syringe.

8. The preparation according to claim **7,** wherein the preparation is a prefilled syringe or an autoinjector pen.

9. A kit, comprising the pharmaceutical composition according to any one of claims 1-6, the preparation according to claim **7** or **8,** and a manual.

10. Use of the pharmaceutical composition according to any one of claims **1-6,** the preparation according to claim **7** or **8,** or the kit according to claim **9** for the manufacture of a medicament for preventing and/or treating an allergic inflammation or an autoimmune disease; wherein
preferably, the allergic inflammation is selected from at least one of asthma, idiopathic pulmonary fibrosis, atopic dermatitis **(AD),** allergic conjunctivitis, allergic rhinitis **(AR)** Netherton syndrome, eosinophilic esophagitis **(EOE),** food allergy, allergic diarrhea, eosinophilic gastroenteritis, allergic bronchopulmonary aspergillosis **(ABPA),** allergic fungal sinusitis, rheumatoid arthritis, chronic obstructive pulmonary disease **(COPD),** systemic sclerosis, keloid, ulcerative colitis, chronic sinusitis **(CRS)** and nasal polyp, chronic eosinophilic pneumonia, eosinophilic bronchitis, **Churg-Strauss** syndrome, eosinophilia, eosinophilic granulomatosis with polyangiitis, inflammatory bowel disease, urticaria, systemic mastocytosis, skin mastocytosis, and recurrent idiopathic angioedema;
optionally, the autoimmune disease is selected from: diabetes, myasthenia gravis, gastritis, pemphigus, primary biliary cirrhosis, multiple sclerosis, lupus, colitis, rheumatoid, psoriasis, and thyroid disease;
optionally, the use comprises administering the medicament in combination with one or more additional therapeutic agents, the additional therapeutic agents being selected from, but not limited to: immunosuppressant (e.g., corticosteroid, nonsteroidal glucocorticoid receptor agonist, leukotriene **D4** antagonist, leukotriene **B4** antagonist, **A2A** agonist, **A2B** antagonist, dopamine receptor agonist, pirfenidone, nintedanib, or **avB6** antagonist), bronchodilator (e.g., **β-2** adrenergic receptor agonist, muscarinic antagonist, short-acting **β2** receptor agonist, long-acting **β2** receptor agonist, short-acting anticholinergic drug, methylxanthine-based drug, and long-acting anticholinergic drug), an additional cytokine or cytokine receptor antagonist or antibody (e.g., **IL-13** antagonist, **IL-6** antagonist, **IL-1, IL-33, IL-25** or **TNF-α** antagonist, anti-**IgE** antibody, anti-**IL-13** antibody, anti**-IL31R** antibody, anti**-IL13** antibody, anti-endoglin antibody, anti**-IL1b** antibody, another anti**-TSLP** antibody, or anti-**hTSLPR** antibody), antibiotic, radiotherapy, leukotriene antagonist (e.g., montelukast, zafirlukast, or pranlukast), **PDE4** inhibitor (such as roflumilast or **xanthene**), antihistamine, or cough suppressant; and
optionally, the antibody or the antigen-binding fragment is administered simultaneously, separately, or sequentially with the additional therapeutic agents.

11. A method for preventing and/or treating an allergic inflammation or an autoimmune disease, comprising administering to a subject in need thereof a prophylactically or therapeutically effective amount of the pharmaceutical composition according to any one of claims **1-6,** the preparation according to claim **7** or **8,** or the kit according to claim **9;** wherein
preferably, the allergic inflammation is selected from at least one of asthma, idiopathic pulmonary fibrosis, atopic dermatitis **(AD),** allergic conjunctivitis, allergic rhinitis **(AR) Netherton** syndrome, eosinophilic esophagitis **(EOE),** food allergy, allergic diarrhea, eosinophilic gastroenteritis, allergic bronchopulmonary aspergillosis **(ABPA),** allergic fungal sinusitis, rheumatoid arthritis, chronic obstructive pulmonary disease **(COPD),** systemic sclerosis, keloid, ulcerative colitis, chronic sinusitis **(CRS)** and nasal polyp, chronic eosinophilic pneumonia, eosinophilic bronchitis, Churg-Strauss syndrome, eosinophilia, eosinophilic granulomatosis with polyangiitis, inflammatory bowel disease, urticaria, systemic mastocytosis, skin mastocytosis, and recurrent idiopathic angioedema;
optionally, the autoimmune disease is selected from: diabetes, myasthenia gravis, gastritis, pemphigus, primary biliary cirrhosis, multiple sclerosis, lupus, colitis, rheumatoid, psoriasis, and thyroid disease;
optionally, the method comprises a step of administering one or more additional therapeutic agents in combination, the additional therapeutic agents being selected from, but not limited to: immunosuppressant (e.g., corticosteroid, nonsteroidal glucocorticoid receptor agonist, leukotriene **D4** antagonist, leukotriene **B4** antagonist, **A2A** agonist, **A2B** antagonist, dopamine receptor agonist, pirfenidone, nintedanib, or **avB6** antagonist), bronchodilator (e.g., **β-2** adrenergic receptor agonist, muscarinic antagonist, short-acting **β2** receptor agonist, long-acting **β2** receptor agonist, short-acting anticholinergic drug, methylxanthine-based drug, and long-acting anticholinergic drug), an additional cytokine or cytokine receptor antagonist or antibody (e.g., **IL-13** antagonist, **IL-6** antagonist, **IL-1, IL-33, IL-25** or **TNF-α** antagonist, anti-**IgE** antibody, anti-**IL-13** antibody, anti**-IL31R** antibody, anti-**IL13** antibody, anti-endoglin antibody, anti-**IL1b** antibody, another anti-**TSLP** antibody, or anti-**hTSLPR** antibody), antibiotic, radiotherapy, leukotriene antagonist (e.g., montelukast, zafirlukast, or pranlukast), **PDE4** inhibitor (such as roflumilast or **xanthene),** antihistamine, or cough suppressant; and
optionally, the pharmaceutical composition, the preparation, or the kit antibody, and the therapeutic agent are administered simultaneously, separately, or sequentially.
